# EUROPÄISCHE PATENTSCHRIFT

(11) **EP 0 820 757 B1**
(45) Veröffentlichungstag und Bekanntmachung des Hinweises auf die Patenterteilung: **17.03.1999**
(21) Anmeldenummer: 97111145.5
(22) Anmeldetag: 03.07.1997
(51) Int. Cl.: A61K 7/06, A61K 7/09

(54) **Mittel zur dauerhaften Verformung von menschlichen Haaren**
Composition for permanent waving of human hair
Composition pour l'ondulation permanente des cheveux humains

(30) Priorität: 26.07.1996 DE 19630262
(43) Veröffentlichungstag der Anmeldung: 28.01.1998
(73) Patentinhaber: GOLDWELL GmbH, D-64297 Darmstadt (DE)
(72) Erfinder: Bräutigam, Ina, 64285 Darmstadt (DE); Rose, Burkhard, 64297 Darmstadt (DE); Nöcker, Bernd, Dr., 64372 Ober-Ramstadt (DE); Schneider, Jörg, 64347 Griesheim (DE)

(56) Entgegenhaltungen:
- EP-A- 0 714 654

## Beschreibung

Die vorliegende Erfindung betrifft ein Mittel zum Verformen von menschlichen Haaren, d.h. ein Dauerwellmittel, das eine ausgezeichnete Wellwirkung besitzt, insbesondere eine gleichmäßige Dauerwelle mit elastischen Locken und guter Kämmbarkeit ergibt, auf die Haarstruktur auch bei mehrfacher Anwendung keinerlei schädigende, sondern vielmehr eine konditionierende Wirkung ausübt und einen ausdrucksvollen Haarglanz hervorruft.

Die Dauerwellung erfordert bekanntlich zwei Behandlungsschritte:
Die reduktive Spaltung der Cystin-Disulfidbrücken des Haares durch Einwirkung eines Reduktionsmittels und die anschließende Neutralisierung bzw. Fixierung durch Aufbringung eines Oxidationsmittels, wodurch die Cystin-Disulfidbrücken wiederhergestellt werden.

Das überwiegend eingesetzte Reduktionsmittel ist auch heute noch Thioglykolsäure, insbesondere als Ammoniumsalz, obwohl zahlreiche andere Thio-Verbindungen für diesen Zweck vorgeschlagen wurden, die sich jedoch in der Praxis nicht durchgesetzt haben.

Die Thioglykolat enthaltenden Zusammensetzungen werden üblicherweise bei einem pH-Wert zwischen 8 und 10, insbesondere 8,5 bis 9,5, eingesetzt, was bei wiederholter, zeitlich nahe zusammenliegender Anwendung zu Haarschädigungen führen kann.

Man hat bereits versucht, diese Nachteile durch die Schaffung sogenannter "saurer Dauerwellmittel" zu überwinden, deren Anwendungs-pH-Wert bei etwa 6,8 bis 7,8, d.h. um den Neutralpunkt herum, liegt. Der in diesen Zusammensetzungen meistbenutzte reduzierende Wirkstoff ist der Thioglykolsäuremonoglycerinester. Es hat sich jedoch gezeigt, daß diese Substanz bei manchen Benutzerinnen hautreizend, insbesondere sensibilisierend, wirkt, so daß auch diese Lösung nicht optimal ist.

Es wurde nunmehr gefunden, daß diese Nachteile überwunden werden können und ein Dauerwellmittel auf Basis mindestens einer reduzierenden organischen Thioverbindung und/oder eines anorganischen Sulfits, das eine gleichmäßige Wellwirkung besitzt und die Haarstruktur in keiner Weise schädigt, sondern dem dauergewellten Haar Glanz und eine gute Kämmbarkeit verleiht, dadurch hergestellt werden kann, wenn man diesem Mittel mindestens ein Organopolysiloxan, nämlich ein gegebenenfalls quaterniertes Aminoalkyl-, insbesondere Aminopropyl-dimethylpolysiloxan/Polyethyloxazolin-Copolymerisat der Formel 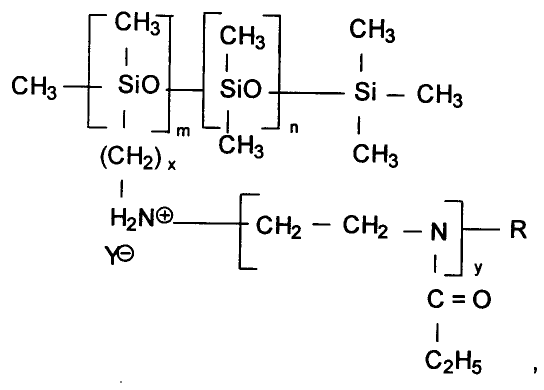 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten. R eine C₁-C₁₂-Alkyl- oder Arylgruppe, insbesondere eine Methyl-, Ethyl- oder Benzylgruppe, und Y⁻ ein Anion darstellen, zusetzt, vorzugsweise in einer Menge von 0,01 bis 5 Gew.-%, insbesondere 0,05 bis 1 Gew.-%. bezogen auf die Gesamtmenge des Mittels,

Die Herstellung der erfindungsgemäß bevorzugt zum Einsatz gelangenden Aminoalkyldimethylpolysiloxan/Polyethyloxazolin-Pfropfcopolymerisate ist in der EP-A 640 643 beschrieben und erfolgt entsprechend dem folgenden Schema:

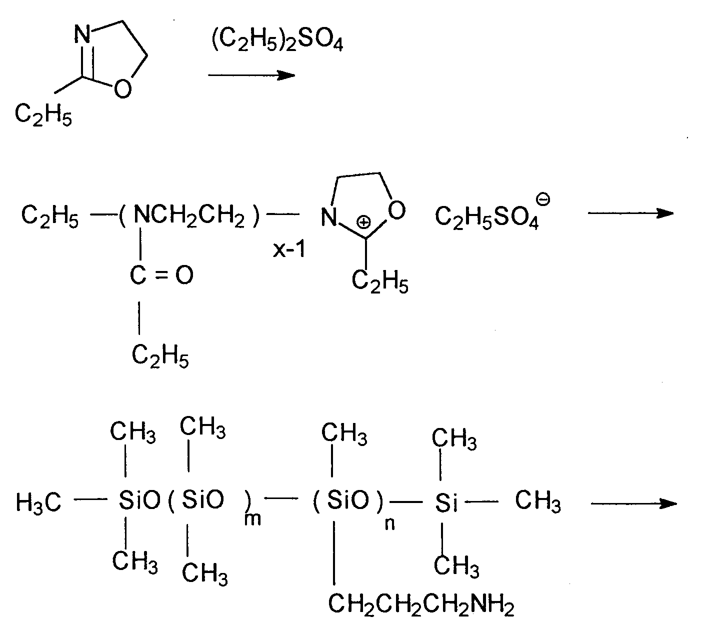

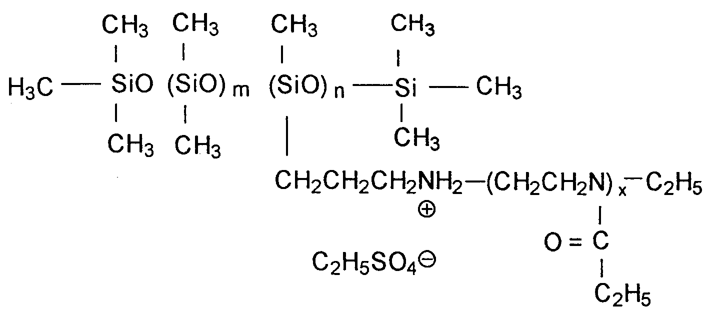

Das Anion Y⁻ der allgemeinen Formel kann selbstverständlich auch ein anderes als das Ethylsulfat-Anion des obengenannten Beispiels sein, d.h. die Quaternierung kann auch mit Methylchlorid, Dimethylsulfat, Benzylchlorid, Dodecylbromid etc. erfolgen.

Ein besonders bevorzugtes Pfropfcopolymerisat der dargestellten Art weist ein Gesamtmolekulargewicht von etwa 50 000 bis etwa 500 000, vorzugsweise etwa 80 000 bis etwa 300 000, insbesondere etwa 100 000 Dalton auf, wobei das Molgewicht des Oxazolin-Segments etwa 2 500 bis etwa 7 500, vorzugsweise etwa 4 000 bis etwa 6 000, insbesondere etwa 5 000 Dalton/Segment beträgt, d.h. der Molanteil bei 20 Einheiten/Molekül liegt. Der bevorzugte Si-Gehalt beträgt, entsprechend der Elementaranalyse, etwa 50 %.

Besonders geeignet sind die unter den Bezeichnungen A-1, A-2 und A-3 auf den Seiten 12 bis 13 der EP-A 640 643 beschriebenen Organopolysiloxane. Der Anteil der Pfropfcopolymerisate in den erfindungsgemäßen Haarbehandlungsmitteln liegt bei etwa 0,05 bis 5, vorzugsweise 0,1 bis 2,5, insbesondere 0,5 bis 1,5 Gew.-% der Zusammensetzung.

Aus der EP-A 714 654 ist es bereits bekannt, diquaternäre Organopolysiloxane in Dauerwellmitteln einzusetzen. Diese Verbindungen stehen mit den erfindungsgemäß verwendeten Organopolysiloxanen in keinem engeren strukturellen Zusammenhang.

Die erfindungsgemäßen Dauerwellmittel enthalten mindestens eine reduzierende organische Thioverbindung und/oder ein anorganisches Sulfit. Bevorzugt sind Thioglykolsäure und Thiomilchsäure sowie deren Salze, insbesondere die Ammonium- und Ethanolaminsalze.

Weitere einsetzbare Thioverbindungen sind vor allem Cystein bzw. dessen Hydrochlorid, Monocystein, Cysteamin, N-Acetylcystein, Thioglycerin, Ethandiolmonothioglykolat, 1,2-Propylenglykolmonothioglykolat (vgl. auch WO-A 93/1791), 1,3-Propandiolmonothioglykolat bzw. das daraus resultierende Isomerengemisch, 1,3-Butandiol- und 1,4-Butandiolmonothioglykolat bzw. deren Isomerengemische, Ethandiolmonothiolactat, 1,2-Propandiol- und 1,3-Propandiolmonothiolactat und deren Isomerengemische, 1,3-Butandiol- und 1,4-Butandiolmonothiolactat und deren Isomerengemische, Polyethylenglykol- wie Di-, Tri- und Tetraethylenglykolmonothioglykolate und -monothiolactate, Polypropylenglykol- wie Di-, Tri- und Tetrapropylenglykolmonothiolactate und -monothioglykolate, Glycerinmonothiolactat und weitere Thiosäuren und deren Ester sowie Gemische derselben.

Der Gesamtgehalt an Reduktionsmitteln in den erfindungsgemäßen Zusammensetzungen beträgt üblicherweise 2,5 bis etwa 15 Gew.-%, berechnet auf freie Thioglykolsäure als Bezugssubstanz.

Als organische Sulfite sind insbesondere Alkalisulfite und -hydrogensulfite geeignet.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.

Bevorzugte Alkalisierungsmittel im Rahmen der Erfindung sind Ammoniumcarbamat, Ammoniak und/oder Ammonium(bi)carbonat. Es wird die Einstellung eines pH-Wertes im Bereich zwischen etwa 6,5 und etwa 9,5, vorzugsweise etwa 7 bis 8,5, angestrebt.

Die erfindungsgemäß zum Einsatz kommenden Dauerwellmittel enthalten vorzugsweise auch Tenside. Deren Anteil liegt bei etwa 0,1 bis etwa 10, insbesondere etwa 1 bis etwa 5 Gew.-%, der das Reduktionsmittel enthaltenden Zusammensetzung.

Sowohl bei den in den Reduktionsmittel-Zusammensetzungen als auch bei den in den Fixiermitteln eingesetzten Tensiden handelt es sich vorzugsweise um die bekannten anionaktiven Produkte, die gegebenenfalls auch in Kombination mit nicht-ionischen Tensiden zum Einsatz gelangen.

Geeignete anionische Tenside sind besonders die bekannten Alkylethersulfate und -carbonsäuren, insbesondere in Form ihrer Alkalisalze, sowie Eiweiß-Fettsäure-Kondensate.

Geeignete nichtionische Tenside sind insbesondere C₈-C₁₈-Fettalkoholpolyglykolether, Fettsäurepolyglykolester, Fettsäurealkanolamide, Aminoxide und vor allem C₈-C₁₈-Alkylpolyglukoside.

Es können auch amphotere Tenside wie die bekannten Betaine und Amidobetaine sowie, insbesondere in kationischen Fixierungen, kationaktive Tenside wie quaternäre Ammoniumverbindungen eingesetzt werden.

Ein weiterer wünschenswerter Bestandteil der erfindungsgemäß verwendeten Reduktionsmittel-Zusammensetzungen ist ein C₃-C₆-Alkandiol bzw. dessen Ether, insbesondere Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 5 bis etwa 10 Gew.-% der Reduktionsmittel-Zusammensetzung.

Neben den C₃-C₆-Alkandiolen bzw. deren Ethern können zusätzlich auch Monoalkohole wie Ethanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Besonders bevorzugte zusätzliche Bestandteile sind anionische, kationische, nichtionische und amphotere Polymere, insbesondere kationische Polymere, vorzugsweise in einer Menge von etwa 0,25 bis etwa 5, insbesondere etwa 0,5 bis 2,5 Gew.-% der Gesamtzusammensetzung des Wellmittels.

Geeignete Polymere sind insbesondere solche des Typs "Polyquaternium" nach dem "CTFA International Cosmetic Ingredient Dictionary", 4th Ed.

Die erfindungsgemäß eingesetzten Mittel können selbstverständlich alle in Dauerwellmitteln üblichen Stoffe enthalten, auf deren detaillierte Aufzählung hier verzichtet wird, und als (wäßrige) Lösungen, Emulsionen, Cremes, Schäume etc. vorliegen.

Zur Vermeidung von Wiederholungen wird hierzu auf den Stand der Technik verwiesen, wie er beispielsweise in "Ullmann's Encyclopedia of Industrial Chemistry", Vol. A12 (1986), S. 588 bis 591, sowie insbesondere in der Monographie von K. Schrader, "Grundlagen und Rezepturen der Kosmetika", 2. Aufl. (1989, Hüthig Verlag), S. 823 bis 840, sowie in dem Übersichtsartikel von D. Hollenberg et al. in "Seifen-Öle-Fette-Wachse" 117 (1991), S. 81 - 87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Falls erwünscht, kann vor dem Auftrag des Reduktionsmittels noch ein Vorbehandlungsmittel appliziert werden, wie es beispielsweise in der DE-A 37 40 926 beschrieben ist. Nach dem Aufbringen dieses Vorbehandlungsmittels wird das Haar aufgewickelt und die Reduktionsmittel-Zusammensetzung aufgetragen. Nach etwa 15- bis 30-minütiger Einwirkung und Spülung erfolgt die Fixierung mit Behandlung durch die üblichen und aus dem Stand der Technik hinreichend bekannten Peroxid- oder Bromat-Zusammensetzungen.

Ebenso kann selbstverständlich eine an sich bekannte Zwischenbehandlung zwischen Reduktions- und Neutralisationsphase erfolgen.

Die folgenden Beispiele dienen der Illustration der Erfindung.

### Beispiel 1

| Dauerwelle für Naturhaar | |
|---|---|
| Ammoniumthioglykolat (60%ig) | 21,5 (Gew.-%) |
| Ammoniumhydrogencarbonat | 5,0 |
| Chlorophyllin, 6%ig | 0,1 |
| Lösungsvermittler (Polyoxyethylenderivat) | 0,8 |
| Harnstoff | 1,0 |
| 1,2-Propandiol | 4,0 |
| Alkyldimethylpolysiloxan/Polyethyloxazolin-Pfropfcopolymerisat (Organopolysiloxan A-1 der EP-A 640 643) | 0,4 |
| Cocoamidopropylbetain | 1,0 |
| Entschäumer, Trübungsmittel, Farbstoff | q.s. |
| Wasser | @ 100.0 |
| Ammoniak zur Einstellung auf | pH 8,5 |

Die Zusammensetzung wurde auf gewickeltes Haar aufgebracht, 20 Minuten bei etwa 30 bis 40° C behandelt, anschließend gespült und mit einer 3%igen Wasserstoffperoxidlotion fixiert. Nach Entfernung der Wickler und nochmaliger Spülung wurde getrocknet.

Es wurde ein gleichmäßig und ausdrucksvoll gewelltes Haar erhalten, das einen angenehmen Glanz und einen weichen Griff aufweist und sich ausgezeichnet kämmen läßt.

Weglassen des Pfropfcopolymerisats führte zu einer deutlich weniger gleichmäßigen und ausdrucksvollen Wellung mit verringertem Glanz, rauherem Griff und schlechterer Kämmbarkeit.

### Beispiel 2

| Saure Dauerwelle Zusammensetzung A: | |
|---|---|
| Ammoniumhydrogencarbonat | 1,0 (g) |
| Kationisches Polymer (Polyquaternium-2) | 0,5 |
| Nichtionischer Lösungsvermittler | 0,8 |
| Alkyldimethylpolysiloxan/Polyethyloxazolin-Pfropfcopolymerisat (Organopolysiloxan A-2 der EP-A 640 643) | 0,1 |
| Cocamidopropylbetain | 1,5 |
| Entschäumer, Trübungsmittel, Parfum | q.s. |
| Wasser | @ 75,0 |
| Ammoniak auf | pH 9,4 |

| Zusammensetzung B: | |
|---|---|
| Glycerinmonothioglykolat, 75%ig | 25,0 (g) |

Unmittelbar vor der Anwendung wurden beide Zusammensetzungen vermischt, die erhaltene Mischung (pH 7,1) auf das Haar aufgebracht und in der in Beispiel 1 beschriebenen Weise dauergewellt und fixiert.

Die erhaltene Dauerwelle war deutlich gleichmäßiger verformt als eine im Halbseitenversuch mit einer Vergleichslösung ohne Pfropfcopolymerisat durchgeführte Dauerwellung und wies auch einen weicheren und lockeren Griff, besseren Glanz und eine verbesserte Kämmbarkeit auf.

### Beispiel 3

| Dauerwelle für Naturhaar Zusammensetzung A: | |
|---|---|
| Ammoniumhydrogencarbonat | 4,5 (g) |
| Kationisches Polymer (Polyquaternium-4) | 1,0 |
| Nichtionischer Lösungsvermittler | 0,8 |
| 1,2-Propandiol | 1,0 |
| Organopolysiloxan/Polyethyloxazolin-Pfropfcopolymerisat (Organopolysiloxan A-1 der EP-A 640 643) | 0,1 |
| Cocamidopropylbetain | 1,0 |
| Entschäumer, Trübungsmittel, Parfüm | q.s. |
| Wasser | @ 75,0 |
| Ammoniak zur Einstellung auf | pH 8,6 |

| Zusammensetzung B: | |
|---|---|
| Ammoniumthioglykolat, 70ig | 18,0 (g) |
| Thiomilchsäure | 1,0 |
| Cystein . HCl | 2,0 |
| 1,2-Propandiol | 0,5 |
| Wasser | @ 28,0 |
| Ammoniak zur Einstellung auf | pH 5,5 |

Beide Zusammensetzungen wurden getrennt in eine Zweikammerdose abgepackt und unmittelbar vor der Anwendung vermischt.

Die erhaltene Mischung (pH 7,4) wurde auf das Haar aufgebracht und die Dauerwellung und Fixierung, wie in Beispiel 1 beschrieben, durchgeführt.

Es wurde eine gleichmäßige ausdrucksvolle Dauerwelle erhalten; das Haar zeigte satten Glanz, angenehmen, weichen Griff und war gut kämmbar.

### Beispiel 4

| Dauerwelle für gefärbtes Haar Zusammensetzung A: | |
|---|---|
| Ammoniumhydrogencarbonat | 3,5 (g) |
| Kationisches Polymer (Polyquaternium-6) | 0,5 |
| Nichtionischer Lösungsvermittler | 0,8 |
| Ethoxydiglycol | 1,5 |
| C₁₂-C₁₄-Alkylpolyglucosid (P.D.= ∼ 1,5) | 1,0 |
| Organopolysiloxan/Polyethyloxazolin-Pfropfcopolymer (Organopolysiloxan A-3 der EP-A 640 643) | 1,0 |
| Parfum, Trübungsmittel, Farbstoff | q.s. |
| Wasser | @ 72,0 |
| Ammoniak zur pH-Einstellung auf | pH 8,3 |

| Zusammensetzung B: | |
|---|---|
| Ammoniumthioglykolat, 70%ig | 13,0 (g) |
| Thiomilchsäure | 0,5 |
| Cystein . HCl | 1,0 |
| Glycin | 0,5 |
| 1,2-Propandiol | 1,0 |
| Wasser | @ 28,0 |
| Ammoniak zur pH-Einstellung auf | pH 5,4 |

Die Zusammensetzungen A und B wurden in einer Zweikammerdose getrennt abgepackt.

Bei der Vermischung, unmittelbar vor dem Dauerwellvorgang, wurde ein pH-Wert von 7,25 erhalten.

Das Produkt ergab, nach dem üblichen Dauerwell- und Fixiervorgang, eine noch bessere Dauerwelle als das nach Beispiel 3.

## Patentansprüche

1. Mittel zur dauerhaften Verformung von menschlichen Haaren, enthaltend
a) mindestens eine reduzierend wirkende organische Thioverbindung und/oder ein anorganisches Sulfit und
b) mindestens ein gegebenenfalls quaterniertes Aminoalkyldimethylpolysiloxan/Polyethyloxazolin-Copolymerisat der Formel 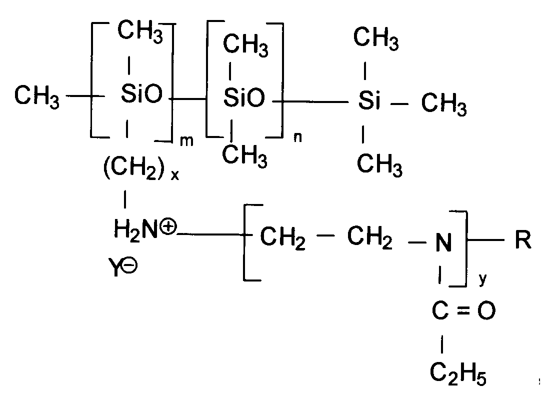 worin m und n jeweils ganze Zahlen von 20 bis 10 000, insbesondere 50 bis 7000, vor allem 100 bis 5000, x eine Zahl zwischen 1 und 5, vorzugsweise 3, und y eine Zahl von 5 bis 30 bedeuten, R eine C₁-C₁₂-Alkyl- oder Arylgruppe, insbesondere eine Methyl-, Ethyl- oder Benzylgruppe, und Y⁻ ein Anion darstellen.

2. Mittel nach Anspruch 1, enthaltend 0,05 bis 5 Gew.-%. berechnet auf die Gesamtzusammensetzung, des Organopolysiloxans.

3. Mittel nach Anspruch 1 oder 2, enthaltend 0,1 bis 2,5 Gew.-% des Organopolysiloxans, berechnet auf die Gesamtzusammensetzung des Mittels.

## Claims

1. Composition for permanent waving of human hair, containing
a) at least one reducing organic thio compound and (or) an inorganic sulfite; and
b) an optionally quaternized aminoalkyl dimethyl polysiloxane/polyethyloxazoline copolymer of the formula 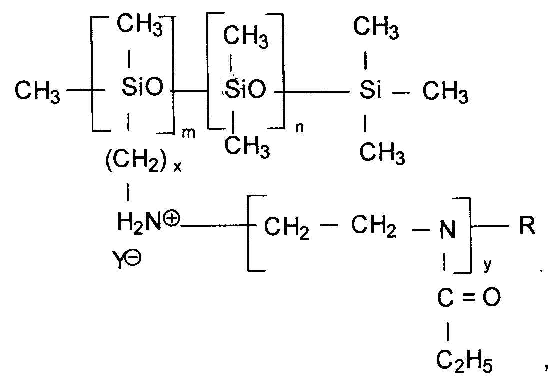 wherein m and n are whole numbers from 20 to 10,000, particularly from 50 to 7000, optimally from 100 to 5000, x is a number from 1 to 5, preferably 3, and y is a number from 5 to 30, R stands for a C₁-C₁₂-alkyl or aryl group,. particularly a methyl, ethyl or benzyl group, and Y⁻ is an anion.

2. Composition according to claim 1, containing 0.05% to 5% by wt. organopolysiloxane, calculated to the total composition.

3. Composition according to claim 1 or 2, containing 0.1% to 2.5% by wt. organopolysiloxane, calculated to the total composition.

## Revendications

1. Agent pour la mise en forme durable de cheveux humains, contenant
a) au moins un composé thio organique à effet réducteur et/ou un sulphite inorganique et
b) au moins un copolymérisat aminoalkyldiméthylpolysiloxane/polyéthyloxazoline éventuellement quaternisé de formule, 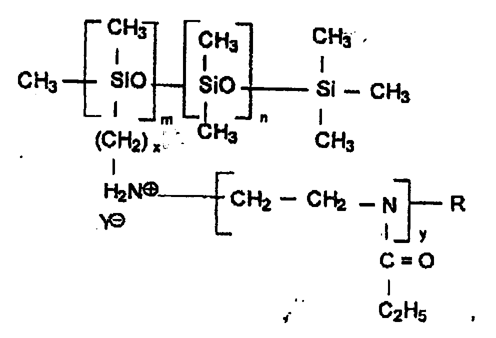 dans laquelle m et n représentent respectivement des nombres entiers de 20 à 10 000, en particulier de 50 à 7 000, surtout de 100 à 5 000, x un nombre compris entre 1 et 5, de préférence 3 et y un nombre de 5 à 30, R représente un groupe alkyle en C₁ à C₁₂ ou aryle, en particulier un groupe méthyle, éthyle ou benzyle, et Y⁻ un anion.

2. Agent selon la revendication 1, contenant 0,05 à 5 % en poids de l'organopolysiloxane, rapporté à la composition totale.

3. Agent selon la revendication 1 ou 2, contenant 0,1 à 2,5 % en poids de l'organopolysiloxane, rapporté à la composition totale de l'agent.
